# EUROPÄISCHE PATENTANMELDUNG

(11) **EP 1 031 559 A1**
(43) Veröffentlichungstag der Anmeldung: **30.08.2000**
(21) Anmeldenummer: 00100900.0
(22) Anmeldetag: 18.01.2000
(51) Int. Cl.: C07C 263/04

(54) **Verfahren zur Herstellung von Isocyanaten durch Spaltung von Urethanen**

(30) Priorität: 23.02.1999 DE 19907648
(71) Anmelder: BASF AKTIENGESELLSCHAFT, 67056 Ludwigshafen (DE)
(72) Erfinder: Dernbach, Matthias, Dr., 69221 Dossenheim (DE); Dittlinger, Wolfgang, 67133 Maxdorf (DE); Schönfelder, Hendrik, Dr., 68159 Mannheim (DE); Henningsen, Michael, Dr., 67227 Frankenthal (DE); Pfeffinger, Joachim, 67063 Ludwigshafen (DE)

(57) **Zusammenfassung**

Verfahren zur Herstellung von Isocyanaten durch Spaltung von Urethanen bei einer Temperatur von 200 bis 600 °C in einem Reaktor, dadurch gekennzeichnet, daß man während der Spaltung mindestens einen Alkohol in den Reaktor einspeist.

## Beschreibung

Gegenstand der Erfindung ist ein Verfahren zur Herstellung von Isocyanaten durch Spaltung von Urethanen bei einer Temperatur von 200 bis 600 °C in einem Reaktor. Des weiteren bezieht sich die Erfindung auf die derart erhältlichen Isocyanate.
Die Herstellung von Isocyanaten durch thermische Spaltung der entsprechenden Urethane ist bekannt und vielfach beschrieben. Derartige Verfahren bieten allgemein den Vorteil, daß zur Herstellung der Isocyanate kein Phosgen benötigt wird.
So wird in EP-A 566 925 ein mehrstufiges kontinuierliches Verfahren zur phosgenfreien Herstellung von Isocyanaten beschrieben. Dabei wird in der ersten Verfahrensstufe, der sogenannten Urethanisierung, Amin mit Harnstoff und einem Alkohol zu einem Carbamidsäureester ("Urethan") umgesetzt.

Danach werden der nichtumgesetzte Alkohol und leichtflüchtige Nebenprodukte, wie Dialkylcarbonat, aus dem Reaktionsgemisch abgetrennt, aufgearbeitet und teilweise in die Reaktionsstufe der Urethanherstellung zurückgeführt. Die verbleibende, überwiegend aus Urethan bestehende Mischung wird geteilt, eine Teilmenge destillativ gereinigt und das Destillat mit der nicht gereinigten Teilmenge der Urethanmischung vereinigt. Diese Urethanmischung wird in der sogenannten Urethan-Spaltung thermisch in Isocyanat und Alkohol gespalten. Der ungespaltene Anteil wird zusammen mit entstandenen Nebenprodukten in die Stufe der Urethanherstellung zurückgeführt.

EP-A 78 005 beschreibt die Herstellung von Isocyanaten durch thermische Spaltung von Urethanen in Gegenwart von Kohlenstoff, bevorzugt in einem gerührten Kohlenstoffbett oder einem Wirbelbett. Als Wirbelgas wird Stickstoff, gegebenenfalls zusammen mit gegenüber Isocyanaten inerten Verbindungen als Lösungs- oder Strippmittel. Die Zugabe von inerten Verbindungen zum Reaktionsgemisch ist allgemein unerwünscht, da diese Substanzen in der Regel an späterer Stelle mit einigem Aufwand aus dem Isocyanat abgetrennt werden müssen.

EP-A 555 628 offenbart Verfahren zur Herstellung von Isocyanaten durch thermische Spaltung von Carbamaten in Gegenwart von gesinterten Oxiden, wobei Stickstoff als Trägergas eingesetzt wird.

Aufgabe der Erfindung war es, ein Verfahren zur Herstellung von Isocyanaten durch thermische Spaltung der entsprechenden Urethane zu entwickeln, das eine hohe Ausbeute liefert und bei dem die Zugabe weiterer Verbindungen, die nicht zu den Edukten oder Produkten gehören, vermieden werden kann.

Die Aufgabe konnte überraschenderweise dadurch gelöst werden, daß man während der Spaltung mindestens einen Alkohol, insbesondere als Strippgas, Wirbelgas und/oder Trägergas, in den Reaktor einspeist. Dies bedeutet, daß zusätzlich zu dem während der Spaltung entstehenden Alkohol weitere Alkoholmengen während der Spaltung zugeführt, d.h. in den Reaktor eingespeist werden.

Isocyanate, insbesondere aromatische Isocyanate, reagieren sehr leicht mit Alkoholen (Houben-Weyl, "Methoden der organischen Chemie", Sauerstoff-Verbindungen III, 1952, S.131). Diesen Umstand macht man sich bei der Hauptnutzung der Isocynate - der Herstellung von Polyurethanen - durch Umsetzung von Polyisocyanaten mit Polyalkoholen zunutze. Der Fachmann, der die Isocyanate mit möglichst hoher Ausbeute aus der Spaltung der Urethane erhalten wollte, hätte somit Alkohole während und nach der Spaltung weitgehend vermieden und während der Spaltung entstehenden Alkohol rasch aus dem Produktgemisch entfernt. Dies ist der Grund, warum in den bekannten Schriften die Spaltung in Gegenwart von inerten Gasen, Lösungsmitteln oder Strippmedien durchgeführt werden, nicht aber in Gegenwart von gegenüber Isocyanaten reaktiven Verbindungen wie Alkoholen. Es war daher nicht zu erwarten, daß durch die erfindungsgemäße Zugabe von Alkohol während der Spaltung Isocyanate in hoher Ausbeute erhalten werden konnten.

In dem erfindungsgemäßen Verfahren können übliche Urethane, bevorzugt Diurethane, zur Spaltung eingesetzt werden. Diese Urethane basieren üblicherweise auf der allgemein bekannten Umsetzung von Aminen, vorzugsweise Di- oder Polyamin, besonders bevorzugt Diaminen, mit Harnstoff und mindestens einem Alkohol.

Die Herstellung des Urethans, die üblicherweise bei einer Temperatur von 160 bis 300 °C und einem Druck von 0,1 bis 60 bar durchgeführt wird, ist beispielsweise in der EP-A 566 925, Spalten 6 bis 9 beschrieben.

Als Alkohole zur Herstellung der Urethane eignen sich prinzipiell alle aliphatischen Alkohole. Vorzugsweise werden solche ausgewählt, deren Siedepunkte hinreichend weit vom Siedepunkt der Isocyanate liegen, um eine optimale Trennung zu gewährleisten.
Besonders bevorzugt werden Methanol, Ethanol, Propanol, Isopropanol, n-Butanol und/oder iso-Butanol zur Herstellung des Urethans eingesetzt.

Als Amine werden bevorzugt 2, 4- und/oder 2, 6-Toluylendiamin (TDA), 2, 2'-, 2, 4'- und/oder 4, 4'-Diphenylmethandiamin (MDA), 1, 6-Hexamethylendiamin (HDA), 1-Amino-3, 3, 5-trimethyl-5-aminomethyl-cyclohexan (im Folgenden auch als Isophorondiamin, IPDA bezeichnet), 1, 5- und/oder 1, 8-Diaminonaphthalin, 4, 4'-Diaminodiphenyl, 1, 3- und/oder 1, 4-Diaminobenzol, 2, 4- und/oder 2, 6-Hexahydrotoluylendiamin und/oder 4, 4'-, 2, 4'- und/oder 2, 2'-Dicyclohexylmethandiamineingesetzt. Die Strukturen der eingesetzten Amine determinieren die Strukturen der nach der thermischen Spaltung erhältlichen Isocyanate. Besonders bevorzugt basieren die eingesetzten Urethane auf 2, 4- und/oder 2, 6-Toluylendiamin (TDA), 2, 2'-, 2, 4'- und/oder 4, 4'-Diphenylmethandiamin (MDA), 1, 6-Hexamethylendiamin (HDA), Isophorondiamin (IPDA) und/oder 1, 5-Diaminonaphthalin als Amin-Komponente und Methanol, Ethanol, Propanol, Isopropanol, n-Butanol und/oder iso-Butanol, insbesondere n-Butanol als Alkohol. Insbesondere werden demnach die folgenden Diurethane zur Spaltung eingesetzt: 2, 4- und/oder 2, 6-Toluylendibutylurethan, 2, 4- und/oder 2, 6-Toluylendiethylurethan, 2, 4- und/ oder 2, 6-Toluylendipropylurethan, 2, 4- und/oder 2, 6-Toluylendimethylurethan, 1, 5-Naphthylendibutylurethan, 1, 5-Naphthylendiethylurethan, 1, 5-Naphthylendipropylurethan, 1, 5-Naphthylendimethylurethan, 4, 4'-, 2, 4'- und/oder 2, 2'-Diphenylmethandibutylurethan, 4, 4'-, 2, 4'- und/oder 2, 2'-Diphenylmethandiethylurethan, 4, 4'-, 2, 4'- und/oder 2, 2'-Diphenylmethandipropylurethan, 4, 4'-, 2, 4'-und/oder 2, 2'-Diphenylmethandimethylurethan, 1, 6-Hexamethylendibutylurethan, 1, 6-Hexamethylendiethylurethan, 1, 6-Hexamethylendipropylurethan, 1, 6-Hexamethylendimethylurethan, Isophorondibutylurethan Isophorondiethylurethan, Isophorondipropylurethan und/oder Isophorondimethylurethan wobei auch Mischungen der genannten Urethane prinzipiell zur Spaltung eingesetzt werden können.

Besonders bevorzugt werden entsprechend die folgenden Isocyanate durch thermische Spaltung der entsprechenden Diurethane hergestellt: 2, 4- und/oder 2, 6-Toluylendiisocyanat (TDI), 2, 2'-, 2, 4'-und/oder 4, 4'-Diphenylmethandiisocyanat (MDI), 1, 6-Hexamethylendiisocyanat (HDI), 1-Amino-3, 3, 5-trimethyl-5-aminomethyl-cyclohexan (Isophorondiisocyanat, IPDI) und/oder 1, 5- Diisocyanatonaphthalin (NDI).

Als Alkohol, der erfindungsgemäß während der Spaltung der Urethane in den Reaktor zugeführt wird, eigenen sich allgemein bekannte Alkohole, bevorzugt Monoalkohole, besonders bevorzugt (cyclo)aliphatische Monoalkohole, insbesondere die Alkohole, die bei der Herstellung der Urethane zur Umsetzung mit den Aminen und dem Harnstoff eingesetzt wurden. Der Einsatz der gleichen Alkohole, die bereits zur Herstellung der Urethane verwendet wurden, bietet den Vorteil, daß die Zugabe von weiteren Verbindungen, die nicht zu den Edukten oder Produkten während des Prozesses gehören, vermieden werden könne, da die Alkohole, die während der thermischen Spaltung zugeführt werden, denen entsprechen, die während der Spaltung freigesetzt werden. Die zugeführten Alkohole können somit zusammen mit den bei der Spaltung freigesetzten Alkoholen von den Isocyanaten abgetrennt und gegebenenfalls erneut als während der thermischen Spaltung eingesetzt werden. Besonders bevorzugt werden Methanol, Ethanol, Propanol, Isopropanol, n-Butanol und/oder iso-Butanol als Alkohol verwendet.

Das molare Verhältnis von zu spaltendem Urethan zu dem Alkohol, der erfindungsgemäß in den Reaktor zugeführt wird, beträgt üblicherweise 100 : 1 bis 0, 01 : 1, bevorzugt 1 : 1 bis 1 : 20, besonders bevorzugt 1 : 5 bis 1 : 15.

Das erfindungsgemäße Verfahren kann in allgemein üblichen Reaktoren, beispielsweise Rührkesseln, Rohrreaktoren, Kolonnen, Rührkesselkaskaden oder Wirbelbehältern kontinuierlich oder diskontinuierlich, bevorzugt in der Gasphase, bei Temperaturen von 200 bis 600°C, bevorzugt 250 bis 450°C und Drücken von üblicherweise 1 mbar bis 1, 5 bar, bevorzugt 1 mbar bis 1 bar erfolgen. Bevorzugt liegt der Alkohol, d.h. der eingespeiste und/oder der durch die Spaltung des Urethans entstehende Alkohol im Reaktor gasförmig vor. Besonders bevorzugt wird der Alkohol, der erfindungsgemäß während der Spaltung in den Reaktor eingeführt wird, in gasförmigem Zustand, bevorzugt mit einer Temperatur von 200 bis 600 °C, in den Reaktor eingespeist. Dabei kann das zu spaltende Urethan flüssig oder gasförmig, bevorzugt gasförmig im Reaktor vorliegen. Das Urethan wird üblicherweise in flüssigem Zustand bei einer Temperatur , die knapp, bevorzugt 1 bis 20 °C, oberhalb der Schmelztemperatur des entsprechenden Urethans liegt, dem Reaktor zugeführt. Der Alkohol wird bevorzugt als Trägergas einsetzt.

Besonders bevorzugt führt man die Spaltung in einer Wirbelschicht durch. Als Wirbelgut werden dabei übliche Materialien in Form von beispielsweise Pulvern oder Preßlingen, z.B. Kugeln, Ringen, Zylindern oder Tabletten, mit einer bevorzugten Korngröße von 0, 01 bis 10 mm, besonders bevorzugt 50 bis 500µm verwendet. Die Wirbelschichtanlagen beinhalten üblicherweise einen beispielsweise zylinderförmigen Wirbelbehälter, dessen Innendurchmesser zur Länge (Höhe) im allgemeinen 1:0, 5 bis 1:10, vorzugsweise 1:1 bis 1:7 beträgt, mit einem Wirbelbett, dem Anströmboden für das Wirbelgas, insbesondere die erfindungsgemäß zugeführten Alkohole, der beispielsweise aus einer Frittenplatte, einem Rundloch- oder Glockenboden oder einem Conidur-Feinlochblech bestehen kann, einer Düse für die Edukt-, d.h. der Urethanzufuhr und der Produkt- und Abgasabfuhr, die gegebenenfalls über einen im Wirbelbehälter befindlichen Filter oder über Zyklone erfolgen kann, gegebenenfalls einer mechanischen Zerkleinerungsvorrichtung für das Wirbelgut und gegebenenfalls Heizflächeneinbauten. Die Urethane werden bevorzugt dampfförmig, gelöst oder in geschmolzener Form, besonders bevorzugt gasförmig in die Wirbelschicht eingedüst.

Als Wirbelgas wird erfindungsgemäß bevorzugt der bei der Spaltung einzuspeisende Alkohol verwendet, wobei der Alkohol gegebenenfalls in Mischung mit weiteren Verbindungen verwendet werden kann, beispielsweise mit Wasser oder inerten Substanzen, z.B. Stickstoff, Helium, Kohlendioxid, (cyclo)aliphatische und/oder aromatische, gegebenenfalls substituierte Kohlenwasserstoffe mit üblicherweise einem Siedepunkt kleiner 250°C °C, beispielsweise Decan, Undecan, Dodecan, Tridecan, Tetradecan, Naphthalin, Toluol, Benzol, 1, 2-, 1, 3- und/oder 1, 4-Dimethylbenzol, Chlorbenzol, aliphatische und/oder aromatische Ketone, beispielsweise Cyclohexanon. Bevorzugt wird das Wirbelgas durch den Anströmboden und das zu spaltende Urethan durch eine seitliche Düse, die sich in der unteren Hälfte der Wirbelschicht befindet, in den Wirbelbehälter eingeführt.

Als Wirbelgut werden bevorzugt Kohlenstoff und/oder gegebenenfalls gesinterte Oxide enthaltend Bor, Aluminium, Silizium, Zinn, Blei, Antimon, Zink, Yttrium, Lanthan, Titan, Zirkonium, Niob, Wolfram und/oder Eisen sowie gegebenenfalls Magnesium, Calzium und/oder Phosphor verwendet. Besonders bevorzugt wird als Wirbelgut Kohlenstoff und/oder ein Steatit, besonders bevorzugt Steatit mit der allgemeinen Formel Mg₃[SiO₁₀(OH)₂] eingesetzt. Die Verwendung des Steatits bietet den Vorteil, daß während des Prozesses gebildete polymere Belegungen in der Wirbelschicht oder an dem Wirbelbehälter durch Ausbrennen entfernt werden können.

Die erfindungsgemäße Spaltung der Urethane kann somit bevorzugt derart erfolgen, daß man kontinuierlich getrennt oder gemeinsam Diurethane und Alkohole, der Alkohol bevorzugt gasförmig, in eine Wirbelschicht, bevorzugt mit Steatit als Wirbelgut, einführt, Isocyanat und Alkohol kontinuierlich entfernt und den Alkohol zumindest teilweise in die Wirbelschicht zurückführt.

Die Spaltprodukte, d.h. das Isocyanat und der Alkohol, werden bevorzugt kontinuierlich aus dem Reaktor entfernt und beispielsweise durch fraktionierte Kondensation getrennt. Der Alkohol kann, wie bereits beschrieben, zumindest teilweise, gegebenenfalls nach ausreichender Reinigung, in den Reaktor, in dem die Spaltung durchgeführt wird, zurückgeführt werden. Das Isocyanat als Produkt kann z.B. durch Destillation gereinigt werden.

Nach dem erfindungsgemäßen Verfahren können Isocyanate in hervorragender Ausbeute unter technisch in einfacher weise zu realisierenden Bedingungen hergestellt werden.

Isocyanate sind wichtige industrielle Chemikalien zur Produktion von Polyurethanen, Pflanzenschutzmitteln und Pharmaka.

Die Erfindung soll an nachfolgenden Beispielen näher beschrieben werden.

### Beispiel 1

In einen von außen elektrisch auf 400°C Innentemperatur beheizten zylindrischen Wirbelschichtreaktor (Durchmesser: 10 cm, Höhe: 50 cm) wurden jeweils 31 Wirbelgut eingefüllt. Als Wirbelgut wurde entweder Steatit, Korngröße 150-350 µm (Bezugsquelle: Massemühle Wagner) oder Acetylenkoks mit einer Korngröße von 200-350 µm verwendet.

Das entsprechende über einen Wärmetauscher auf eine Temperatur von 400°C vorgeheizte Wirbelgas wurde über eine Bodenplatte in die Wirbelschicht eingedüst. Bei Verwendung von Alkoholen wurde der Alkohol über einen Vorverdampfer verdampft und dann der Wirbelschicht zugeführt.

Ein geschmolzenes Gemisch aus (4-Methyl-1, 3-Phenylen)biscarbaminsäuredibutylester (2, 4-Toluylendibutylurethan oder auch 2, 4-Butyl-TDU genannt) und (2-Methyl-1, 3-Phenylen)biscarbaminsäuredibutylester (2, 6-Toluylendibutylurethan oder auch 2, 6-Butyl-TDU genannt) Isomerengemisch mit einem Isomerenverhältnis von 2, 4-Butyl-TDU : 2, 6-Butyl-TDU von 80:20 wurde über eine Pumpe und eine seitlich angebrachte Düse in den Wirbelschichtreaktor eingedüst.

Das Spaltgas wurde direkt in gasförmigem Zustand am Reaktorausgang hinsichtlich des NCO-Gehalts analysiert.

Die Reaktionsbedingungen und Ergebnisse sind in Tabelle 1 aufgeführt.

| Beispiel | 1 | 2 | 3 | 4 | 5 | 6 |
|---|---|---|---|---|---|---|
| Wirbelgut | A | A | A | S | S | S |
| Wirbelgutmenge [kg] | 2, 6 | 2, 6 | 2, 6 | 4, 3 | 4, 3 | 4, 3 |
| Wirbelgas | N₂ | CO₂ | BuOH | N₂ | BuOH | N₂, H₂O |
| Wirbelgasmenge [Nm³/h] | 0, 755 | 0, 755 | 0, 755 | 1, 5 | 1, 5 | 1, 5 N₂ + 5, 8g H₂O/h |
| TDU-Feed [g/h] | 946 | 943 | 942 | 522 | 538 | 531 |
| Belastung pro Liter Wirbelgut [kg TDU/1*h] | 0, 32 | 0, 31 | 0, 31 | 0, 17 | 0, 18 | 0, 18 |
| NCO-Ausbeute von theoretischer Ausbeute [%] | 55, 5 | 47, 7 | 66, 4 | 92, 7 | 96, 4 | 93, 7 |
| Die Beispiele 3 und 5 stellen erfindungsgemäße Beispiele dar. | | | | | | |

| | | | | | | |
|---|---|---|---|---|---|---|
| BuOH: n-Butanol | | | | | | |
| S: Steatit | | | | | | |
| A: Acetylenkoks | | | | | | |

Anhand der Beispiele wird deutlich, daß entgegen der Erwartung eine Spaltung der Urethane in Gegenwart von Alkohol, der während der Spaltung zugeführt wird, im Vergleich zu inerten Verbindungen als Trägergas zu höheren Ausbeuten führt. Dies ist umso überraschender, als der Fachmann erwarten würde, daß das Isocyanat als Spaltprodukt mit dem zugeführten Alkohol bevorzugt zum Urethan zurückreagiert.

## Patentansprüche

1. Verfahren zur Herstellung von Isocyanaten durch Spaltung von Urethanen bei einer Temperatur von 200 bis 600 °C in einem Reaktor, dadurch gekennzeichnet, daß man während der Spaltung mindestens einen Alkohol in den Reaktor einspeist.

2. Verfahren nach Anspruch 1, dadurch gekennzeichnet, daß man die Spaltung in der Gasphase durchführt.

3. Verfahren nach Anspruch 1, dadurch gekennzeichnet, daß man die Spaltung in einer Wirbelschicht durchführt.

4. Verfahren nach Anspruch 2, dadurch gekennzeichnet, daß man den Alkohol als Trägergas einsetzt.

5. Verfahren nach Anspruch 3, dadurch gekennzeichnet, daß man den Alkohol als Wirbelgas einsetzt.

6. Verfahren nach Anspruch 3, dadurch gekennzeichnet, daß man Kohlenstoff und/oder Steatit mit der allgemeinen Formel Mg₃[SiO₁₀(OH)₂] als Wirbelgut in der Wirbelschicht verwendet.

7. Verfahren nach Anspruch 1, dadurch gekennzeichnet, daß man Methanol, Ethanol, Propanol, Isopropanol, n-Butanol und/oder iso-Butanol als Alkohol verwendet.

8. Verfahren nach Anspruch 1, dadurch gekennzeichnet, daß man Diurethane spaltet.

9. Verfahren nach Anspruch 1, dadurch gekennzeichnet, daß man kontinuierlich getrennt oder gemeinsam Diurethane und Alkohole in eine Wirbelschicht einführt, Isocyanat und Alkohol kontinuierlich entfernt und den Alkohol zumindest teilweise in die Wirbelschicht zurückführt.

10. Isocyanate erhältlich durch ein Verfahren gemäß einem der Ansprüche 1 bis 9.
